# EUROPÄISCHE PATENTANMELDUNG

(11) **EP 0 704 427 A1**
(43) Veröffentlichungstag der Anmeldung: **03.04.1996**
(21) Anmeldenummer: 95114634.9
(22) Anmeldetag: 18.09.1995
(51) Int. Cl.: C07C 241/02, C07C 243/10

(54) **Verfahren zur Herstellung von Alkylhydrazinsalzen**

(30) Priorität: 29.09.1994 DE 4434847
(71) Anmelder: BAYER AG, D-51368 Leverkusen (DE)
(72) Erfinder: Eichinger, Wolfram, Dr., D-51061 Köln (DE); Fiege, Helmut, Dr., D-51373 Leverkusen (DE)

(57) **Zusammenfassung**

Alkylhydrazinsalze werden in technischem Maßstab und mit guten Ergebnissen hergestellt, indem man Hydrazin, ein Alken und eine starke Protonensäure in Gegenwart von Wasser miteinander umsetzt, wobei man Hydrazin und die Protonensäure vorlegt, das Alken zudosiert, die im Reaktionsgefäß vorhandene flüssige und gasförmige Phase bei 75 bis 150°C intensiv miteinander mischt, danach das Reaktionsgemisch auf Temperaturen unter 60°C abkühlt, das ausgefallene Alkylhydrazinsalz abtrennt, die Mutterlauge erneut mit Hydrazin und starker Protonensäure versetzt, anschließend wieder Alken eindosiert und so den nächsten Reaktionszyklus durchführt.

## Beschreibung

Die vorliegende Erfindung betrifft ein im technischen Maßstab mit guten Ergebnissen durchführbares Verfahren zur Herstellung von Alkylhydrazinsalzen aus Hydrazin, einer Protonensäure und einem Alken.

Alkylhydrazine sind wichtige Zwischenprodukte zur Herstellung von Arznei- und Pflanzenschutzmitteln, z.B. zur Herstellung von N-t-Alkyl-1,2-diacylhydrazinen, die insektizide Wirkungen haben.

Aus der JP-OS 63-72 661 (= JP-Anm. 61-218 729) ist bekannt, daß man Alkylhydrazine aus Hydrazin und einem Alken in Gegenwart einer anorganischen Säure herstellen kann. Die Reaktion ist nur in kleinem Maßstab beschrieben (0,1 Mol Alken), und das Alkylhydrazin wurde nicht isoliert, sondern nur im Reaktionsgemisch analysiert. Es werden nicht unerhebliche Mengen von Dialkylhydrazinen als Nebenprodukte erhalten.

Gemäß der US-PS 4 954 655 wird ganz ähnlich verfahren. Auch hier ist die Reaktion nur in kleinem Maßstab beschrieben (höchstens 0,16 Mol Hydrazin). Das hergestellte Alkylhydrazin wurde auch hier nicht isoliert, sondern das Reaktionsgemisch mit einer starken Base und Benzoylchlorid versetzt und letztlich N-t-Alkyl-1,2-diacylhydrazine erhalten.

Bei beiden Verfahren wird das Alken einfach in das Reaktionsgemisch eingeleitet oder ein großer Überschuß davon durch das Reaktionsgemisch geführt.

Bei Versuchen, diese bekannten Verfahren in größerem Maßstab durchzuführen, wurde festgestellt, daß dann keine Reaktion mehr stattfindet, auch wenn man die Reaktionsbedingungen verschärft, z.B. die Temperatur und/oder den Druck erhöht, die Reaktionszeit verlängert oder rührt. Außerdem besteht das Bedürfnis nach einem Verfahren, bei dem Alkylhydrazine in reiner Form anfallen und isoliert werden können, weil nur dann die Herstellung des Alkylhydrazins und seine Weiterverarbeitung zu Arznei- und Pflanzenschutzmitteln an getrennten Orten (z.B. bei verschiedenen Firmen) durchgeführt werden können.

Es wurde nun ein Verfahren zur Herstellung von Alkylhydrazinsalzen der Formel (I) gefunden 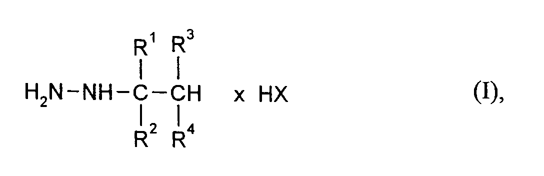
in der
- R¹: für C₁-C₄-Alkyl,
- R²: für C₁-C₄-Alkyl, C₂-C₄-Alkenyl, C₂-C₄-Alkinyl oder Phenyl,
- R³ und R⁴: unabhängig voneinander jeweils für Wasserstoff, C₁-C₄-Alkyl, C₂-C₄-Alkenyl oder C₂-C₄-Alkinyl oder
- R¹ und R² oder R³ und R⁴: gemeinsam mit dem dazwischenliegenden C-Atom für C₅-C₈-Cycloalkyl und
- X: für das Anion einer starken Protonensäure stehen,
aus Hydrazin und einem Alken der Formel 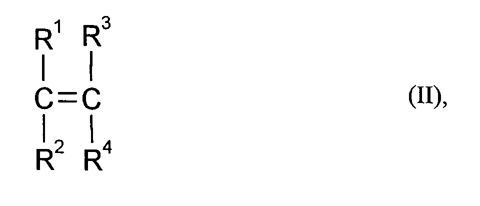
in der
R¹, R², R³ und R⁴ die bei Formel (I) angegebene Bedeutung haben,
in Gegenwart von Wasser und einer starken Protonensäure, das dadurch gekennzeichnet ist, daß man Hydrazin und die Protonensäure vorlegt, das Alken der Formel (II) zudosiert, die im Reaktionsgefäß vorhandene flüssige und gasförmige Phase bei 75 bis 150°C intensiv miteinander vermischt, danach das Reaktionsgemisch auf Temperaturen unter 60°C abkühlt, das ausgefallene Alkylhydrazinsalz der Formel (I) abtrennt, die Mutterlauge erneut mit Hydrazin und starker Protonensäure versetzt, anschließend wieder Alken der Formel (II) eindosiert und so den nächsten Reaktionszyklus durchführt.

Bevorzugt einzusetzende Alkene der Formel (II) sind beispielsweise Isobutylen, 2-Methyl-2-buten, 2-Methyl-1-hexen, 2,3-Dimethyl-buten, 1-Methyl-cyclohexen, Methylencyclohexan und α-Methylstyrol.

In den Formeln (I) und (II) stehen
- R¹: besonders bevorzugt für Methyl oder Ethyl,
- R²: besonders bevorzugt für Methyl, Ethyl, Ethenyl oder Phenyl, insbesondere für Methyl oder Ethyl und
- R³ und R⁴: unabhängig voneinander besonders bevorzugt jeweils für Wasserstoff, Methyl oder Ethyl, insbesondere beide für Wasserstoff.

Ganz besonders bevorzugt wird in das erfindungsgemäße Verfahren Isobutylen eingesetzt und ein Mono-tert.-butylhydrazin-Salz hergestellt.

Als starke Protonensäuren kommen z.B. Fluorwasserstoff, Chlorwasserstoff, Bromwasserstoff, Schwefelsäure, Phosphorsäure und kationische Ionenaustauscher in der H-Form in Frage. Bevorzugt sind Chlorwasserstoff und Schwefelsäure. In Formel (I) steht deshalb X beispielsweise für ein Äquivalent Fluorid, Chlorid, Bromid, Sulfat, Phosphat oder Ionenaustauscherrest.

Die starken Protonensäuren können als solche oder in Form von wäßrigen Lösungen eingesetzt werden. Es können auch Gemische mehrerer starker Protonensäuren eingesetzt werden.

Das Wasser dient als Lösungsmittel für das erfindungsgemäße Verfahren. Es kann in Form von Hydrazinhydrat, wäßrigen Protonensäuren und/oder als solches zugesetzt werden.

Das Hydrazin kann z.B. als solches, als Hydrat, als Hydraziniumsalz der verwendeten Protonensäure und/oder als dessen wäßrige Lösung eingesetzt werden.

Es ist vorteilhaft, die Protonensäure im Überschuß zu Hydrazin einzusetzen, beispielsweise 1,05 bis 1,8 Äquivalente Protonensäure pro Mol Hydrazin.

Das Alken kann, bezogen auf 1 Mol Hydrazin z.B. in einer Menge von 0,5 bis 1,5 Mol eingesetzt werden. Vorzugsweise beträgt diese Menge 0,8 bis 1,2 Mol. Man kann das Alken in flüssiger oder gasförmiger Form einsetzen und der flüssigen und/oder der gasförmigen Phase zudosieren.

Temperaturen im Bereich von 85 bis 110°C sind für die Umsetzung von Hydrazin, Protonensäure und Alken bevorzugt. Der Druck bei dieser Umsetzung kann in weiten Grenzen variiert werden und beispielsweise 1 bis 100 bar betragen. Eine Arbeitsweise unter Druck bzw. in einem geschlossenen Reaktionsgefäß ist im allgemeinen vorteilhaft, um zu einem guten Ergebnis zu kommen.

Es ist ein wesentliches Merkmal der vorliegenden Erfindung, daß man die im Reaktionsgefäß vorhandene flüssige und gasförmige Phase intensiv miteinander vermischt. Rühren mit einem einfachen Rührer ist dazu nicht ausreichend.

Die notwendige intensive Vermischung kann z.B. erreicht werden, indem man, z.B. beginnend bei der Eindosierung des Alkens, die flüssige Phase aus dem Reaktionsgefäß kontinuierlich ganz oder teilweise abpumpt und im Gasraum des Reaktionsgefäßes versprüht. Ebenso kann man die gasförmige Phase aus dem Reaktionsgefäß kontinuierlich ganz oder teilweise abpumpen und der flüssigen Phase in feinverteilter Form wieder zuführen. Es ist vorteilhaft, nach Beendigung der Zugabe des Alkens die intensive Durchmischung noch einige Zeit, z.B. 20 Minuten bis 4 Stunden, fortzuführen oder wenigstens zu rühren. Auf diese Weise erreicht man nahezu quantitative Umsätze des eingesetzten Alkens.

Danach wird das Reaktionsgemisch auf Temperaturen unter 60°C abgekühlt, vorzugsweise auf -10 bis +60°C, insbesondere auf 0 bis 20°C. Dabei fällt zwischen 20 und 40 Gew.-% des im Reaktionsgemisch vorhandenen Alkylhydrazinsalzes der Formel (I) aus. Man kann es z.B. durch Filtration abtrennen und gegebenenfalls mit frischem Wasser nachwaschen.

Obwohl auf diese Weise nur ein geringer Anteil des gebildeten Alkylhydrazinsalzes der Formel (I) isoliert wird kann dessen Herstellung doch auf eine wirtschaftliche Weise erfolgen. Es hat sich nämlich überraschenderweise gezeigt, daß keine unerwünschte Anreicherung von Nebenprodukten erfolgt, wenn man die Mutterlauge nach der Abtrennung des ausgefällten Alkylhydrazinsalzes erneut mit Hydrazin und starker Protonensäure versetzt, anschließend wieder Alken der Formel (II) eindosiert und so den nächsten Reaktionszyklus beginnt. Insgesamt erhält man dann das Alkylhydrazinsalz in Ausbeuten von ca. 90 % der Theorie und in Reinheiten von ca. 99 %.

Bei der Wiederverwendung der Mutterlauge, der auch gegebenenfalls das Waschwasser zugefügt werden kann, ist lediglich darauf zu achten, daß sich das Reaktionsgemisch nicht zu sehr verdünnt. Dies würde z.B. eintreten, wenn man das Waschwasser der Mutterlauge zufügt und/oder das Hydrazin in Form von Hydrazinhydrat und/oder die starke Protonensäure als wäßrige Lösung einsetzt. Eine zu weitgehende Verdünnung kann z.B. durch Abdestillieren von Wasser aus der Mutterlauge und gegebenenfalls aus dem Waschwasser verhindert werden.

Die Rückführung der Mutterlauge kann mindestens 20 mal wiederholt werden.

Die Reaktionsgefäße zur Durchführung des erfindungsgemäßen Verfahrens sollen resistent gegen starke Protonensäuren sein. Sie können z.B. aus Glas, Kunststoff, Tantal oder Tantallegierungen bestehen oder mit Glas oder Emaille beschichtet sein.

Das erfindungsgemäße Verfahren gestattet die Herstellung und Isolierung von Alkylhydrazinsalzen der Formel (I) in technischem Maßstab in guten Ausbeuten und mit hohen Reinheiten.

### Beispiele

### Beispiel 1

In einem 4 l-Rührautoklaven aus Stahl / Emaille wurden 1630 g 37 gew.-%ige wäßrige Salzsäure, 701 g Hydrazinhydrat (= 14 Mol) und 470 g Wasser vorgelegt. Der Reaktor wurde verschlossen und unter Rühren auf 100°C erhitzt. Danach wurden im Verlauf von 5,5 Stunden unter kräftigem Rühren 706 g Isobutylen in feinverteilter Form unter die Flüssigkeitsoberfläche zudosiert und gleichzeitig die Gasphase über eine Gaspumpe partiell abgepumpt und der flüssigen Phase in feinverteilter Form wieder zugeführt. Nach Beendigung der Isobutylen-Zugabe wurde noch 1 Stunde nachgerührt. Dann wurde das Reaktionsgemisch auf +3°C abgekühlt, der ausgefallene Niederschlag abfiltriert und der erhaltene Filterkuchen mit 250 g Wasser gewaschen. Nach dem Trocknen des Filterkuchens lagen 471 g tert.-Butylhydrazin-hydrochlorid in einer Reinheit von über 99 % vor. Das entspricht einer Ausbeute von 28,2 %, bezogen auf Hydrazin.

### Beispiel 2 (zum Vergleich)

Salzsäure, Hydrazinhydrat und Wasser wurden entsprechend Beispiel 1 vorgelegt. Nach dem Verschließen des Reaktionsgefäßes wurde auf 85°C geheizt und unter Rühren innerhalb von 20 Minuten 50 g Isobutylen in den Gasraum zudosiert. Dabei stieg der Druck auf 6 bar. Innerhalb der nächsten 3 Stunden wurde keine Druckabnahme beobachtet. Dann wurde die Temperatur auf 100°C erhöht. Wiederum wurde im Verlauf von 3 Stunden keine Druckabnahme beobachtet. Schließlich wurden die restlichen 656 g Isobutylen zugegeben (15,3 bar) und das Reaktionsgemisch 18 Stunden bei 100°C gerührt. Es erfolgte wiederum keine Druckabnahme. Abschließend wurde das Reaktionsgemisch auf +3°C abgekühlt, wobei keine Niederschlag ausfiel. Es hatte offenbar keine Reaktion stattgefunden.

### Beispiel 3

Beispiel 1 wurde wiederholt, die nach dem Abfiltrieren des Reaktionsgemischs verbleibende Mutterlauge (3215 g) wurde mit dem Waschwasser vereinigt und aus diesem Gemisch 500 g Wasser abdestilliert. Dann wurden die in der ersten Zeile der nachfolgenden Tabelle 1 angegebenen Mengen 37 gew.-%iger wäßriger Salzsäure, Hydrazinhydrat und Isobutylen hinzugefügt und entsprechend Beispiel 1 umgesetzt und aufgearbeitet. Dieser Reaktionszyklus wurde 10 mal wiederholt. Die Einzelheiten sind aus der nachfolgenden Tabelle 1 ersichtlich.

**Tabelle 1**

| Reaktionszyklus | Einsatzmengen (g) | | | | abgetrenntes Produkt (g) trocken |
|---|---|---|---|---|---|
| | Mutterlauge | Salzsäure | Hydrazinhydrat | Isobutylen | |
| 2 | 3215 | 400 | 193 | 236 | 510 |
| 3 | 3081 | 380 | 193 | 241 | 391 |
| 4 | 3147 | 400 | 193 | 236 | 604 |
| 5 | 2996 | 436 | 218 | 260 | 446 |
| 6 | 3162 | 370 | 187 | 232 | 540 |
| 7 | 3107 | 400 | 202 | 249 | 427 |
| 8 | 3251 | 390 | 181 | 201 | 549 |
| 9 | 3074 | 440 | 222 | 271 | 402 |
| 10 | 3252 | 356 | 180 | 213 | 533 |
| 11 | 2966 | 420 | 210 | 255 | 428 |

Nach insgesamt 11 Reaktionszyklen betrug die Gesamtausbeute an trockenem tert.-Butylhydrazin-hydrochlorid 79,3 % der Theorie, bezogen auf insgesamt eingesetztes Hydrazin. Diese Ausbeute wird noch höher, wenn weitere Reaktionszyklen durchgeführt werden.

## Patentansprüche

1. Verfahren zur Herstellung von Alkylhydrazinsalzen der Formel 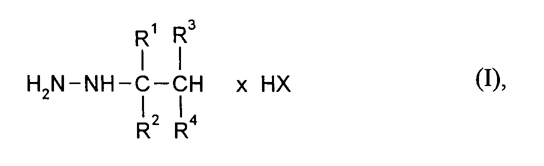 in der
R¹ für C₁-C₄-Alkyl,
R² für C₁-C₄-Alkyl, C₂-C₄-Alkenyl, C₂-C₄-Alkinyl oder Phenyl,
R³ und R⁴ unabhängig voneinander jeweils für Wasserstoff, C₁-C₄-Alkyl, C₂-C₄-Alkenyl oder C₂-C₄-Alkinyl oder
R¹ und R² oder R³ und R⁴ gemeinsam mit dem dazwischenliegenden C-Atom für C₅-C₈-Cycloalkyl und
X für das Anion einer starken Protonensäure stehen,
aus Hydrazin und einem Alken der Formel 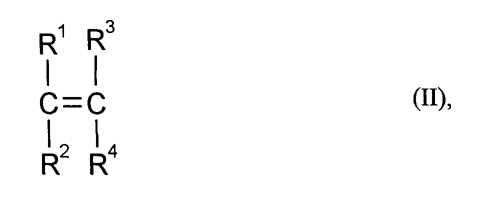 in der
R¹, R², R³ und R⁴ die bei Formel (I) angegebene Bedeutung haben,
in Gegenwart von Wasser und einer starken Protonensäure, dadurch gekennzeichnet, daß man Hydrazin und die Protonensäure vorlegt, das Alken der Formel (II) zudosiert, die im Reaktionsgefäß vorhandene flüssige und gasförmige Phase bei 75 bis 150°C intensiv miteinander vermischt, danach das Reaktionsgemisch auf Temperaturen unter 60°C abkühlt, daß ausgefallene Alkylhydrazinsalz der Formel (I) abtrennt, die Mutterlauge erneut mit Hydrazin und starker Protonensäure versetzt, anschließend wieder Alken der Formel (II) eindosiert und so den nächsten Reaktionszyklus durchführt.

2. Verfahren nach Anspruch 1, dadurch gekennzeichnet, daß man als Alken Isobutylen, 2-Methyl-2-buten, 2-Methyl-1-hexen, 2,3-Dimethyl-buten, 1-Methyl-cyclohexen, Methylencyclohexan oder α-Methylstyrol einsetzt.

3. Verfahren nach Ansprüchen 1 und 2, dadurch gekennzeichnet, daß in den Formeln (I) und (II)
R¹ für Methyl oder Ethyl,
R² für Methyl, Ethyl, Ethenyl oder Phenyl,
R³ und R⁴ unabhängig voneinander für Wasserstoff, Methyl oder Ethyl und
X für ein Äquivalent Fluorid, Chlorid, Bromid, Sulfat, Phosphat oder eines Ionenaustauscherrestes steht.

4. Verfahren nach Ansprüchen 1 bis 3, dadurch gekennzeichnet, daß man als starke Protonensäure, Fluorwasserstoff, Chlorwasserstoff, Bromwasserstoff, Schwefelsäure, Phosphorsäure oder einen kationischen Ionenaustauscher in der H-Form einsetzt.

5. Verfahren nach Ansprüchen 1 bis 4, dadurch gekennzeichnet, daß man pro Mol Hydrazin 1,05 bis 1,8 Mol Protonensäure einsetzt.

6. Verfahren nach Ansprüchen 1 bis 5, dadurch gekennzeichnet, daß man aus dem Reaktionsgefäß die flüssige Phase kontinuierlich ganz oder teilweise abpumpt und im Gasraum des Reaktionsgefäßes versprüht.

7. Verfahren nach Ansprüchen 1 bis 5, dadurch gekennzeichnet, daß aus dem Reaktionsgefäß die gasförmige Phase kontinuierlich teilweise oder ganz abpumpt und der flüssigen Phase in feinverteilter Form wieder zuführt.

8. Verfahren nach Ansprüchen 1 bis 7, dadurch gekennzeichnet, daß man das Reaktionsgemisch auf 0 bis 20°C abkühlt.

9. Verfahren nach Ansprüchen 1 bis 8, dadurch gekennzeichnet, daß man aus der Mutterlauge vor deren Rückführung einen Teil des vorhandenen Wassers abdestilliert.

10. Verfahren nach Ansprüchen 1 bis 9, dadurch gekennzeichnet, daß man Reaktionsgefäße verwendet, die aus Glas, Kunststoff, Tantal oder Tantallegierungen bestehen oder mit Glas oder Emaille beschichtet sind.
